(19) Europäisches Patentamt
European Patent Office
Office européen des brevets

(11) Veröffentlichungsnummer: **0 062 157**
**B1**

(12) # EUROPÄISCHE PATENTSCHRIFT

(45) Veröffentlichungstag der Patentschrift: **27.06.84**

(51) Int. Cl.³: **A 61 K 31/12**

(21) Anmeldenummer: **82101574.0**

(22) Anmeldetag: **02.03.82**

(54) **Arzneimittel zur lokalen Behandlung der Alopecia areata.**

(30) Priorität: **28.03.81 DE 3112374**

(43) Veröffentlichungstag der Anmeldung: **13.10.82 Patentblatt 82/41**

(45) Bekanntmachung des Hinweises auf die Patenterteilung: **27.06.84 Patentblatt 84/26**

(84) Benannte Vertragsstaaten: **BE CH DE FR GB IT LI LU NL SE**

(56) Entgegenhaltungen:
**US - A - 3 366 664**
**US - A - 3 657 348**
**US - A - 3 787 500**

**Die Akte enthält technische Angaben, die nach dem Eingang der Anmeldung eingereicht wurden und die nicht in dieser Patentschrift enthalten sind.**

(73) Patentinhaber: **Happle, Rudolf, Prof. Dr.med., Schildstiege 2, D-4400 Münster-Roxel (DE)**

(72) Erfinder: **Hausen, Björn, Dr., Wilhelmstrasse 4, D-2082 Tornesch (DE)**
Erfinder: **Happle, Rudolf, Prof. Dr., Schildstiege 2, D-4400 Münster-Roxel (DE)**

(74) Vertreter: **Glawe, Delfs, Moll & Partner Patentanwälte, Rothenbaumchaussee 58 Postfach 2570, D-2000 Hamburg 13 (DE)**

**EP 0 062 157 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die Erfindung betrifft Arzneimittel zur lokalen Behandlung der Alopecia areata und anderer Hautkrankheiten. Das Arzneimittel der Erfindung weist einen Gehalt an einem Cyclopropenon der in Anspruch 1 angegebenen allgemeinen Formel auf.

Die Alkylgruppen der allgemeinen Formel bedeuten z. B. Methyl, Ethyl, Propyl, Butyl oder Pentyl. Bevorzugt sind Gruppen mit 12—16 Kohlenstoffatomen mit der Summenformel $C_{12}H_{25}$, $C_{13}H_{27}$, $C_{14}H_{29}$, $C_{15}H_{31}$ und $C_{16}H_{33}$.

Das Cyclopropenon der im Anspruch angegebenen allgemeinen Formel kann auch mit Alkylengruppen mit 3—18, insbesondere 12—16 Kohlenstoffatomen, die 1, 2 oder 3 konjugierte oder isolierte Doppelbindungen aufweisen, substituiert sein. Bevorzugte Substituenten weisen z. B. die folgenden Formeln auf:

— $(CH_2)_7CH = CH(CH_2)_5CH_3$
— $(CH_2)_7CH = CHCH_2CH = CH(CH_2)_2CH_3$
— $(CH_2)_7CH = CHCH_2CH = CHCH = CHCH_3$
— $(CH_2)_7CH = CHCH_2CH = CHCH_2CH = CH_2$

Bei den Alkyl- und Alkenylgruppen als Substituenten für das Cyclopropenon sind geradkettige, d. h. unverzweigte Gruppen bevorzugt.

Von den als Substituenten vorgesehenen Alkoxygruppen sind Methoxy, Ethoxy, Propoxy und Butoxy bevorzugt.

Die Substituenten $R^1$ und $R^2$ können auch aromatische Reste wie Phenyl oder Naphthyl bedeuten, die gegebenenfalls durch Halogen, Alkyl-, Monoalkylamino-, Dialkylamino- und/oder Alkoxygruppen oder mehrere dieser Gruppen substituiert sein können. Dabei bedeutet Halogen insbesondere Fluor, Chlor und Brom; Alkyl bzw. Alkoxy bedeutet hier insbesondere Gruppen mit 1—4 Kohlenstoffatomen.

Gemäß einer bevorzugten Ausführungsform der Erfindung enthält das Arzneimittel Diphenylcyclopropenon als Wirkstoff.

Die Gruppen $R^1$ und $R^2$ können auch verschieden sein; als Beispiel ist hier das 1-Phenyl-2-methoxy-cyclopropenon zu nennen.

Das Arzneimittel der Erfindung kann übliche pharmazeutische Träger und/oder Verdünnungsmittel enthalten. Es kann z. B. in Form einer Lösung eingesetzt werden, insbesondere gelöst in Aceton. Der Wirkstoff kann weiterhin in üblichen Salbenformulierungen oder zusammen mit Filmbildnern verabreicht werden.

Die Konzentration des Wirkstoffs in dem Arzneimittel der Erfindung hängt von der speziellen Sensibilisierung des Patienten ab, sie kann zwischen $10^{-5}$ und 6 Gew.-%, insbesondere 0,01 bis 3 Gew.-% betragen.

Die therapeutische Anwendung des Arzneimittels der Erfindung erfolgt bei der Alopecia areata in der Weise, daß die erkrankten Hautpartien ca. 1mal pro Woche mit dem Arzneimittel bestrichen werden, wobei auf den behandelten Hautpartien eine lokale Allergie erzeugt wird. Bereits nach kurzer Behandlungsdauer läßt sich auf den behandelten Hautpartien ein erneutes Haarwachstum beobachten. Das Arzneimittel ist darüber hinaus auch zur Behandlung anderer Hautkrankheiten (z. B. Viruswarzen) geeignet.

## Patentansprüche

1. Arzneimittel zur lokalen Behandlung der Alopecia areata und anderer Hautkrankheiten, gekennzeichnet durch einen Gehalt an einem Cyclopropenon der allgemeinen Formel

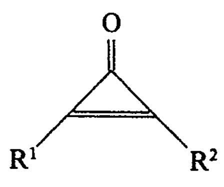

in der die Gruppen $R^1$ und $R^2$ die gleich oder verschieden sein können, Alkylgruppen mit 1—18, insbesondere 12—16 Kohlenstoffatomen, Alkylengruppen mit 3—18, insbesondere 12—16 Kohlenstoffatomen und die 1, 2 oder 3 konjugierte oder isolierte C = C-Bindungen aufweisen können, Alkoxygruppen mit 1—4 Kohlenstoffatomen in dem Akylrest oder aromatische Reste wie Phenyl oder Naphthyl, die gegebenenfalls durch Halogen, Alkyl-, Monoalkylamino-, Dialkylamino- und/oder Alkoxygruppen oder mehrere dieser Gruppen substituiert sein können, bedeuten.

2. Arzneimittel nach Anspruch 1, gekennzeichnet durch einen Gehalt an Diphenylcyclopropenon.

## Claims

1. Medicament for the local treatment of alopecia areata and other skin disorders, characterised by a content of a cyclopropenone of the general formula

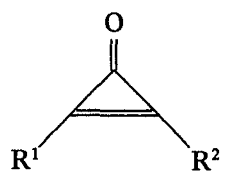

wherein the groups $R^1$ and $R^2$, which may be the same or different, represent alkyl groups having 1—18, in particular 12—16 carbon atoms, alkylene groups having 3—18, in particular 12—16 carbon atoms, and which may have 1, 2 or 3 conjugated or isolated C = C-bonds, alkoxy groups having 1—4 carbon atoms in the alkyl radical or aromatic radicals, such as phenyl or naphthyl, which optionally can be substituted by halogen, alkyl-, monoalkylamino-, dialkylamino- and/or alkoxy groups or several of these groups.

2. Medicament according to claim 1, characterised by a content of diphenylcyclopropenone.

## Revendications

1. Médicament pour le traitement local d'Alopecia areata et d'autres dermatoses, characterisé en ce qu'il contient un cyclopropénone de la formule générale

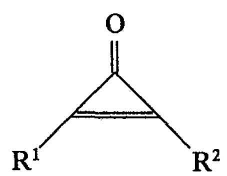

dans laquelle les groupes $R^1$ et $R^2$ qui peut être pareils ou differents, sont des groupes alkyle de 1—18, notamment 12—16 atomes de carbone, des groupes alkényle de 3—18, notamment 12—16 atomes de carbone et qui peut contenir 1, 2 ou 3 doubles liaisons conjugées ou isolées, des groupes alcoxy de 1—4 atomes de carbone dans le radical alkyle ou des radicaux aromatiques comme le phényle ou le naphthyle qui, le cas échéant, peut être substitués par une groupe halogène, alkyle, monoalkylamino, dialkylamino et/ou alcoxy ou par plusieurs de ces groupes.

2. Médicament selon la revendication 1, characterisé en ce qu'il contient de diphénylcyclopropénone.